# EUROPÄISCHE PATENTSCHRIFT

(11) **EP 3 996 792 B1**
(45) Veröffentlichungstag und Bekanntmachung des Hinweises auf die Patenterteilung: **26.03.2025**
(21) Anmeldenummer: 20815779.2
(22) Anmeldetag: 26.11.2020
(51) Int. Cl.: A61M 27/00, A61F 2/94, A61F 2/966, A61F 2/04

(54) **APPLIKATIONSVORRICHTUNG MIT EINER URETERSCHIENE UND EINER EINFÜHRKOMPONENTE ZUM EINFÜHREN DER URETERSCHIENE, EINFÜHRKOMPONENTE UND APPLIKATIONSEINRICHTUNG**
APPLICATION DEVICE WITH A URETERAL STENT AND AN INSERTION COMPONENT FOR INSERTING THE URETERAL STENT, INSERTION COMPONENT AND APPLICATION DEVICE
DISPOSITIF D'APPLICATION AVEC UN STENT URÉTÉRAL ET UN COMPOSANT D'INSERTION POUR L'INSERTION DU STENT URÉTÉRAL, UN COMPOSANT D'INSERTION ET UN DISPOSITIF D'APPLICATION

(30) Priorität: 27.11.2019 DE 102019132160
(43) Veröffentlichungstag der Anmeldung: 18.05.2022
(73) Patentinhaber: Urotech GmbH, 83101 Rohrdorf-Achenmühle (DE)
(72) Erfinder: REITER, Niclas, 83607 Holzkirchen (DE); HOCHBURGER, Tobias, 83022 Rosenheim (DE)
(74) Vertreter: Hofstetter, Schurack & Partner
(86) Internationale Anmeldenummer: PCT/EP2020/083494
(87) Internationale Veröffentlichungsnummer: WO 2021/105278

(56) Entgegenhaltungen:
- US-A1- 2010 160 848
- US-A1- 2015 182 362
- US-B1- 6 258 098

## Beschreibung

Die Erfindung betrifft eine Applikationsvorrichtung, mit einer Ureterschiene, welche einen Ureterschienengrundkörper und einen mittels wenigstens eines Verbindungselements der Ureterschiene mit dem Ureterschienengrundkörper verbundenen Aufnahmekörper umfasst, sowie mit einer Einführkomponente, zum Halten der Ureterschiene und zum Führen der Ureterschiene durch einen Harnleiter eines Patienten entlang eines biegeweichen Führungselements. Weitere Aspekte der Erfindung betreffen eine Einführkomponente für eine derartige Applikationsvorrichtung sowie eine Applikationseinrichtung.

Zum Einsetzen von Ureterschienen wird häufig ein Führungsdraht über eine Harnröhre, auch Urethra genannt, eine Blase und einen Harnleiter, auch Ureter genannt, in eine Niere eines Patienten eingeführt. Über den Führungsdraht kann anschließend die Ureterschiene mithilfe einer Einführhilfe platziert werden. Nach der Positionierung der Ureterschiene wird die Einführhilfe ebenso wie der Führungsdraht entfernt. Das Herausziehen des Führungsdrahtes führt in der Regel zu einer Formänderung, meist eine aufrollende Bewegung, an einem distalen Ende oder an beiden Enden der Ureterschiene. Diese Formänderung verhindert ein ungewolltes Herausrutschen der Ureterschiene und dient somit zu deren Fixierung im Harnsystem des Patienten.

Um eine Zystoskopie zum Entfernen der Ureterschiene zu vermeiden, kann letztere an deren distalem Ende einen Magneten umfassen, welcher mit einem Katheter, insbesondere Tiemann-Katheter, der ein den Magneten anziehendes Ende aufweist, magnetisch verbunden werden kann. Eine zwischen dem Magneten und dem anziehenden Ende des Katheters wirkende Magnetkraft reicht aus, um die über den Magneten mit dem Katheter gekoppelte Ureterschiene anhand des Katheters in kürzester Zeit zu entfernen.

Aus der DE 697 31 027 T2 ist ein Stent zur Implantation in einen Harnleiter zwischen einer Harnblase und einer Niere zur Verhinderung von Rückfluss von Urin in die Niere bekannt. Der Stent umfasst einen länglichen röhrenförmigen Körper mit einem Harnblasen-Endabschnitt und einem Nieren-Endabschnitt, Flüssigkeitspfade im Nieren-Endabschnitt und über mindestens einen Teil des röhrenförmigen Körpers, Mittel am Nieren-Endabschnitt und am Harnblasen-Endabschnitt zur Krümmung der Enden des Stents, so dass sie im Nierenbecken und in der Harnblase gehalten werden, und Mittel zur Unterdrückung des Flusses von Urin aus der Harnblase zu der Niere über den Stent. Der Stent umfasst entweder keinen Durchgang oder einen Durchgang im Harnblasen-Endabschnitt, der nach der Implantation des Stents geschlossen ist, wobei der Harnblasen-Endabschnitt das Unterdrückungsmittel bildet.

Die DE 10 2015 001 505 A1 beschreibt eine Einrichtung, die von außen etwa wie ein Blasenkatheter aussieht, die aber aus einem Mantelschlauchteil und einem Spitzenteil zusammengesetzt ist, das über einen zentral im Mantelschlauchteil liegenden Führungsdraht derart am blasenseitigen Ende des Mantelschlauchteils angeordnet ist, dass durch Betätigung des Führungsdrahtes am Griffelement das Spitzenteil aus dem Mantelschlauchteil translatorisch ausschiebbar und relativ zum Mantelschlauchteil verdrehbar ist, so dass das Spitzenteil in seiner herausgeschobenen Arbeitsposition ein am Führungsdraht befestigtes Greifteil mit elastisch verformbaren Greifarmen freigibt, wobei letztere beim Verdrehen des Greifteils Zugfäden bzw. Fadenschlingen erfassen und aufwickeln, die außen am distalen Endbereich der Doppel-J-Harnleiterschiene angeordnet sind. Aus der US 2015/182362 A1 ist eine Applikationsvorrichtung gemäß dem Oberbegriff des Anspruchs 1 bekannt. Die US 2010/160848 A1 offenbart einen Ureterstent mit einem langgestreckten Element mit einem ersten Abschnitt und zweiten Abschnitt, der mit dem ersten Abschnitt gekoppelt ist. Der erste Abschnitt des länglichen Elements ist konfiguriert, um in einer Niere eines Patienten angeordnet zu werden. Der zweite Abschnitt, der eine Seitenwand aufweist, die ein Lumen definiert, ist so konfiguriert, dass er Fluid von einer ersten Stelle der Seitenwand des zweiten Abschnitts zu einer zweiten Stelle der Seitenwand des zweiten Abschnitts durch Kapillarwirkung abgibt. Der zweite Abschnitt des langgestreckten Elements ist dabei so konfiguriert, dass er in mindestens einem von einer Blase und einem Harnleiter des Patienten angeordnet ist. Die US 6 258 098 B1 zeigt eine Stent-Anordnung, umfassend einen langgestreckten Stent mit einem distalen Ende; ein ferromagnetisches Element mit einer Länge, die wesentlich kürzer als die Länge des Stents ist; und ein Halteseil, dessen einer Teil mit dem ferromagnetischen Element verbunden ist und dessen anderer Teil mit dem distalen Ende des Stents verbunden ist.

Aufgabe der vorliegenden Erfindung ist es, eine Applikationsvorrichtung, eine Einführkomponente sowie eine Applikationseinrichtung der eingangs genannten Art zu schaffen, mittels welchen eine vereinfachte Ausrichtung einer Ureterschiene bei deren Einführen in Richtung einer Niere erfolgen kann.

Diese Aufgabe wird durch den Gegenstand des unabhängigen Patentanspruchs 1 gelöst. Vorteilhafte Weiterbildungen der Erfindung sind durch die Merkmale der abhängigen Patentansprüche, die folgende Beschreibung sowie die Figuren offenbart.

Ein erster Aspekt der Erfindung betrifft eine Applikationsvorrichtung, mit einer Ureterschiene, welche einen Ureterschienengrundkörper und einen mittels wenigstens eines Verbindungselements der Ureterschiene mit dem Ureterschienengrundkörper verbundenen Aufnahmekörper umfasst, sowie mit einer Einführkomponente, zum Halten der Ureterschiene und zum Führen der Ureterschiene durch einen Harnleiter eines Patienten entlang eines biegeweichen Führungselements.

Gemäß der Erfindung ist vorgesehen, dass die Ureterschiene mittels der Einführkomponente in einander entgegengesetzte Bewegungsrichtungen entlang dem biegeweichen Führungselement verlagerbar ist und die Einführkomponente dazu ausgebildet ist, den Ureterschienengrundkörper und den Aufnahmekörper beim Verlagern in den jeweiligen Bewegungsrichtungen zu führen. Dies ist von Vorteil, da die Ureterschiene und damit der Ureterschienengrundkörper sowie der Aufnahmekörper somit gemeinsam anhand der Einführkomponente relativ zu dem biegeweichen Führungselement und in die einander entgegengesetzten Bewegungsrichtungen verschoben werden können, sodass eine besonders genaue Orientierung und damit Ausrichtung der Ureterschiene bei deren Einführen in Richtung der Niere des Patienten ermöglicht ist. Beim Verlagern in den jeweiligen, entgegengesetzten Bewegungsrichtungen kann dementsprechend ein Vor- und Zurückziehen der Ureterschiene in der Harnröhre, der Blase, dem Harnleiter oder der Niere mittels der Einführkomponente erfolgen.

Die Ureterschiene kann auch als Harnleiterschiene bezeichnet werden. Der Ureterschienengrundkörper kann auch als Stent, also als medizinisches Implantat zum Offenhalten von Gefäßen oder Hohlorganen, bezeichnet werden. Die Einführkomponente kann auch als sogenannter "Pusher" bezeichnet werden. Das Führungselement kann vorzugsweise als Führungsdraht, also als Draht zum Führen der Applikationsvorrichtung ausgebildet sein. Denkbar ist prinzipiell auch eine Ausgestaltung des Führungselements als Kunststoffdraht oder als Kunststoffrohr. Das Führungselement kann über eine Harnröhre, auch Urethra genannt, eine Blase und den Harnleiter, auch Ureter genannt, in die Niere eines Patienten eingeführt werden. Die Ureterschiene kann dann zusammen mit der Einführkomponente an dem Führungselement entlang ebenfalls in die Niere eingeführt und damit durch den Harnleiter durchgeführt werden. Die Ureterschiene kann über das biegeweiche Führungselement an der Einführkomponente gehalten werden, wobei das biegeweiche Führungselement hierzu sowohl durch die Ureterschiene als auch durch die Einführkomponente hindurchgeführt werden kann. Zudem umfasst die Einführkomponente eine Ausnehmung zum Einführen und Halten des Aufnahmekörpers. Dies ist von Vorteil, da der Aufnahmekörper in der Ausnehmung besonders platzsparend angeordnet werden kann und mittels der Ausnehmung zudem auf einfache Weise ein etwaiges, ungewünschtes Herausfallen des Aufnahmekörpers vermieden werden kann. Die Ausnehmung kann dabei sozusagen eine Aufnahme für den Aufnahmekörper bilden. Dabei umfasst die Einführkomponente zudem wenigstens zwei Rohrabschnitte, in welche das biegeweiche Führungselement einführbar ist, sowie zumindest einen, die wenigstens zwei Rohrabschnitte miteinander verbindenden Verbindungsbereich, wobei die wenigstens zwei Rohrabschnitte und der zumindest eine Verbindungsbereich die Ausnehmung begrenzen. Dies ist von Vorteil, da die Einführkomponente somit eine Außenkontur aufweist, durch welche die Einführkomponente in besonderem Maße zur Einführung in jeweilige Kanäle des Harnsystems, wie beispielsweise die Harnröhre oder den Harnleiter geeignet ist. Durch die Möglichkeit, das biegeweiche Führungselement in die Rohrabschnitte einzuführen kann die Einführkomponente besonders verliersicher an dem biegeweichen Führungselement entlang und beispielsweise in die Blase des Patienten eingeführt werden, um nur ein Beispiel zu nennen. Die Rohrabschnitte können jeweilige Rohrdurchgangsöffnungen aufweisen, durch welche das biegeweiche Führungselement durchgeführt werden kann. Gemäß der Erfindung ist ein erster Rohrabschnitt der wenigstens zwei Rohrabschnitte als proximales Ende der Einführkomponente ausgebildet, in welches zumindest bei an der Einführkomponente gehaltener Ureterschiene ein dem Aufnahmekörper zugewandtes, distales Grundkörperende des Ureterschienengrundkörpers zumindest bereichsweise eingeführt ist. Dies ist von Vorteil, da durch das Einführen des distalen Grundkörperendes eine besonders verliersichere, lösbare Befestigung des distalen Grundkörperendes und damit des Ureterschienengrundkörpers an der Einführkomponente gegeben ist. Das distale Grundkörperende kann auch als distales Ende des Ureterschienengrundkörpers bezeichnet werden. Insgesamt können also das distale Grundkörperende und das proximale Ende der Einführungskomponente (Pusher) bei an der Einführkomponente gehaltener Ureterschiene miteinander in Kontakt stehen. Als proximales Ende des jeweiligen Elementes der Applikationsvorrichtung wird dabei das jeweils näher zur Körpermitte liegende Ende, als distales Ende das jeweils weiter von der Körpermitte entfernte Element der Applikationsvorrichtung verstanden. Des Weiteren weist der erste, proximale Rohrabschnitt der Einführkomponente eine erste Rohrdurchgangsöffnung auf, deren Durchmesser sich in Richtung des Aufnahmekörpers verringert, derart, dass dieser ungefähr gleich oder gleich einem Durchmesser einer Durchgangsöffnung des Aufnahmekörpers ist. Es besteht aber auch die Möglichkeit, dass im ersten, proximalen Rohrabschnitt der Einführkomponente ein Tubus angeordnet ist, wobei der Tubus eine Tubusdurchgangsöffnung aufweist, deren Durchmesser sich in Richtung des Aufnahmekörpers verringert, derart, dass dieser ungefähr gleich oder gleich einem Durchmesser einer Durchgangsöffnung des Aufnahmekörpers ist. Des Weiteren besteht die Möglichkeit, dass der Tubus eine Tubusdurchgangsöffnung aufweist, deren Durchmesser ungefähr gleich oder gleich einem Durchmesser einer Durchgangsöffnung des Aufnahmekörpers ist. Die genannten Ausführungsformen gewährleisten eine sichere und leichte Durchführung und Beweglichkeit des Führungselementes innerhalb der Durchführöffnungen des Ureterschienengrundkörpers, der Einführkomponente und des Aufnahmekörpers. Durch die entsprechende Anpassung der Durchmesser kann zuverlässig verhindert werden, dass das Führungselement beispielsweise auf die Stirnfläche des Aufnahmekörpers treffen kann und so ein Weiterschieben des Führungselements durch die Einführkomponente und den Aufnahmekörper hindurch verhindert. Schließlich ist ein proximaler Endbereich des ersten, proximalen Rohrabschnitts der Einführkomponente trichterförmig zur Aufnahme des distalen Grundkörperendes ausgebildet. Es besteht auch die Möglichkeit, dass die Trichterform durch einen proximalen Endbereich des in dem ersten, proximalen Rohrabschnitt der Einführkomponente angeordneten Tubus ausgebildet wird. Die Trichterform erleichtert das Andocken wie auch das Festhalten des distalen Ureterschienengrundkörpers an dem proximalen Ende der Einführkomponente.

Die Einführkomponente kann besonders bevorzugt dazu ausgebildet sein, den Ureterschienengrundkörper und den Aufnahmekörper unabhängig von der Verlagerung in die einander entgegengesetzten Bewegungsrichtungen voneinander beabstandet, insbesondere in einem konstanten Abstand zueinander, zu halten. Dadurch kann in vorteilhafter Weise ein etwaiges Kollidieren sowie ein beispielsweise daraus resultierendes Verkippen des Aufnahmekörpers relativ zu dem Ureterschienengrundkörper beim Einführen in Richtung der Niere vermieden werden, wodurch Komplikationen beim Einführen der Ureterschiene vermieden werden können. Mit anderen Worten kann also eine etwaige Relativbewegung zwischen dem Ureterschienengrundkörper und dem Aufnahmekörper beim Verlagern vermieden werden.

Die Einführkomponente, welche auch als Einführhilfe bezeichnet werden kann, kann vorzugsweise einteilig ausgebildet sein, womit eine besonders einfache Handhabbarkeit der Einführkomponente erreicht werden kann. Insbesondere ist dadurch keine zusätzliche Mechanik zum Lösen der Einführkomponente von der Ureterschiene nach deren bestimmungsgemäßer Platzierung im Harnsystem nötig.

In einer vorteilhaften Weiterbildung der Erfindung weist der Aufnahmekörper ferromagnetische Eigenschaften auf. Der Aufnahmekörper kann also mit anderen Worten zumindest bereichsweise als Ferromagnet ausgebildet sein. Damit kann der Aufnahmekörper durch einen Magneten, welcher beispielsweise an einem Katheter, insbesondere Tiemann-Katheter, aufgenommen oder Teil des Katheters sein kann, angezogen werden. Alternativ dazu kann der Aufnahmekörper auch zumindest bereichsweise als Magnetkörper ausgebildet und damit selbst magnetisch sein. Sowohl die magnetische Anziehbarkeit als auch die Ausgestaltung als Magnet erleichtern die Entfernung der Ureterschiene, zumal der Aufnahmekörper dann schnell - infolge von entsprechend wirkenden Magnetkräften - mit dem Katheter (Tiemann-Katheter) verbunden und aus dem Harnsystem entfernt, insbesondere herausgezogen, werden kann.

In einer weiteren vorteilhaften Weiterbildung der Erfindung ist der Ureterschienengrundkörper gemeinsam mit dem Aufnahmekörper anhand der Einführkomponente relativ zu dem biegeweichen Führungselement drehbar. Dies ist von Vorteil, da hierdurch die Orientierung und damit die Ausrichtung der Ureterschiene bei deren Einführen weiter verbessert werden kann. Die Einführkomponente kann insbesondere dazu ausgebildet sein, den Ureterschienengrundkörper und den Aufnahmekörper derart zu halten, dass diese anhand der Einführkomponente synchron, also mit anderen Worten drehversatzfrei, drehbar sind.

In einer weiteren vorteilhaften Weiterbildung der Erfindung ist das wenigstens eine Verbindungselement biegeschlaff oder biegeweich ausgebildet. Dies ist von Vorteil, da der Aufnahmekörper bei einem späteren Entfernen der Ureterschiene besonders leicht in Richtung eines Katheters, insbesondere Tiemann-Katheters, gezogen werden kann, wobei das Verbindungselement dabei unter besonders geringem Kraftaufwand verformt werden kann, wodurch eine magnetische Verbindung zwischen den Aufnahmekörper und dem Katheter auf vereinfachte Weise hergestellt werden kann.

In einer weiteren vorteilhaften Weiterbildung der Erfindung ist das wenigstens eine Verbindungselement bei an der Einführkomponente gehaltener Ureterschiene mittels der Einführkomponente gespannt. Dies ist von Vorteil, da somit die Ureterschiene besonders verliersicher an der Einführkomponente gehalten ist. So kann in besonders vorteilhafter Weise vorgesehen sein, dass die Einführkomponente über das Verbindungselement zwischen dem Aufnahmekörper und dem Ureterschienengrundkörper bereichsweise eingeklemmt ist, wodurch ein unbeabsichtigtes Lösen der Ureterschiene von der Einführkomponente besonders vorteilhaft unterbunden werden kann. Mit anderen Worten kann also das Verbindungselement über die Einführkomponente unter mechanischer Spannung gehalten sein, wenn die Ureterschiene an der Einführkomponente gehalten ist.

In einer weiteren vorteilhaften Weiterbildung der Erfindung ist das wenigstens eine Verbindungselement als Fadenelement oder als Drahtelement ausgebildet. Dies ist von Vorteil, da das Verbindungselement dadurch besonders wenig Raum beansprucht und damit das Einführen der Ureterschiene in Richtung der Niere nicht unnötig erschwert. Darüber hinaus ist durch die Ausgestaltung des Verbindungselements als Fadenelement oder als Drahtelement eine besonders einfache Verformung des Verbindungselements möglich, sodass das Verbindungselement eine magnetische Koppelung zwischen dem Aufnahmekörper und einem Katheter zum Entfernen der Ureterschiene nicht behindert.

**In** einer weiteren vorteilhaften Weiterbildung der Erfindung ist das zumindest bereichsweise eingeführte distale Grundkörperende bei an der Einführkomponente gehaltener Ureterschiene mittels des Führungselements an dem ersten Rohrabschnitt in einer Einführstellung haltbar, wobei das distale Grundkörperende dazu ausgebildet ist, sich bei von dem Ureterschienengrundkörper gelöstem Führungselement selbständig von der Einführstellung in eine Grundstellung des distalen Grundkörperendes zu verformen und sich dadurch aus dem ersten Rohrabschnitt heraus zu bewegen. Das Führungselement kann also das distale Grundkörperende in der Einführstellung halten. Dies ist von Vorteil, da das distale Grundkörperende in dessen Einführstellung besonders widerstandsarm beispielsweise in die Harnröhre eingeführt werden kann und sich ohne weitere Hilfsmittel und damit sozusagen selbständig von der Einführstellung in die Grundstellung verformen kann, wenn das Führungselement von dem Ureterschienengrundkörper gelöst ist, also beispielsweise aus dem Ureterschienengrundkörper herausgezogen und dadurch von dem Ureterschienengrundkörper getrennt, insbesondere beabstandet, ist. Infolge des Verformens von der Einführstellung in die Grundstellung kann sich das distale Grundkörperende selbständig aus dem ersten Rohrabschnitt heraus bewegen. Das Führungselement kann mit anderen Worten also das zumindest bereichsweise eingeführte distale Grundkörperende bei an der Einführkomponente gehaltener Ureterschiene in der Einführstellung halten, wobei das distale Grundkörperende dazu ausgebildet ist, sich bei von dem Ureterschienengrundkörper gelöstem Führungselement selbständig von der Einführstellung in die Grundstellung zu verformen und sich dadurch aus dem ersten Rohrabschnitt heraus zu bewegen.

In der Grundstellung kann das distale Grundkörperende bevorzugt eine größere radiale Erstreckung aufweisen, als in der Einführstellung. Dadurch kann ein unerwünschtes Herausgleiten oder Verrutschen des beispielsweise durch den Harnleiter geführten Ureterschienengrundkörpers in dessen Grundstellung vermieden werden. Der Ureterschienengrundkörper kann entsprechend sicher im Harnleiter gehalten werden. Im Gegensatz zur Einführstellung kann das distale Grundkörperende in der Grundstellung vorzugsweise eingedreht sein. Gleiches kann auch für ein dem distalen Grundkörperende gegenüberliegendes, proximales Grundkörperende des Ureterschienengrundkörpers gelten. Mit anderen Worten kann vorgesehen sein, dass sich auch das dem distalen Grundkörperende gegenüberliegende, proximale Grundkörperende selbstständig von dessen Einführstellung in dessen Grundstellung verformen kann. Auch das proximale Grundkörperende kann in dessen Grundstellung vorzugsweise eingedreht sein. Insbesondere können die beiden einander gegenüberliegenden Grundkörperenden des Ureterschienengrundkörpers in deren jeweiliger Grundstellung jeweils j-förmig eingedreht sein. In deren jeweiliger Einführstellung können die einander gegenüberliegenden Grundkörperenden jeweils im Vergleich zur Grundstellung gestreckt und vorgespannt sein. Das distale Grundkörperende und/oder das proximale Grundkörperende kann/können sich zusammenfassend durch Lösen des Führungselements von dem Ureterschienengrundkörper von der Einführstellung in die Grundstellung verformen. Mittels des Führungselements kann/können das distale Grundkörperende und/oder das proximale Grundkörperende in der Einführstellung vorgespannt gehalten werden. Das distale Grundkörperende und/oder das proximale Grundkörperende kann/können sich zusammenfassend beim Verformen von der Einführstellung in die Grundstellung vorzugsweise eindrehen.

In einer weiteren vorteilhaften Weiterbildung der Erfindung ist das distale Grundkörperende dazu ausgebildet, bei dessen Herausbewegen aus dem ersten Rohrabschnitt und durch das Verformen von der Einführstellung in die Grundstellung den Aufnahmekörper über das wenigstens eine Verbindungselement aus der Ausnehmung heraus zu ziehen. Dies ist von Vorteil, da der Aufnahmekörper somit ohne weitere Hilfsmittel durch das Verformen des distalen Grundkörperendes und über das Verbindungselement von der Einführkomponente gelöst werden kann. Beim Herausbewegen aus dem ersten Rohrabschnitt kann sich das distale Grundkörperende während dessen Verformung von der Einführstellung in die Grundstellung an dem ersten Rohrabschnitt abstützen und dadurch über das wenigstens eine Verbindungselement den Aufnahmekörper aus der Ausnehmung heraus bewegen.

Insbesondere kann das biegeweiche Führungselement zunächst zumindest aus dem ersten Rohrabschnitt herausgezogen werden, wodurch sich das distale Grundkörperende von der Einführstellung in die Grundstellung verformen kann und sich dadurch selbständig aus dem ersten Rohrabschnitt herausbewegen kann. Dabei kann sich das distale Grundkörperende an dem ersten Rohrabschnitt abstützen und dabei den Aufnahmekörper über das Verbindungselement aus der Ausnehmung herausziehen. Das distale Grundkörperende kann sich also durch die Verformung vom Einführzustand in den Grundzustand automatisch aus dem ersten Rohrabschnitt herausbewegen.

In weiteren vorteilhaften Ausgestaltungen der erfindungsgemäßen Applikationsvorrichtung kann der erste, proximale Rohrabschnitt der Einführkomponente in einem proximalen Endbereich eine Verengung aufweisen, wobei ein Innendurchmesser der Verengung kleiner ist als ein Außendurchmesser des distalen Endes des Ureterschienengrundkörpers. Es besteht auch die Möglichkeit, dass der erste, proximale Rohrabschnitt der Einführkomponente in einem proximalen Endbereich sich verjüngend ausgebildet ist, derart, dass der Innendurchmesser der Verengung kleiner ist als der Außendurchmesser des distalen Endes des Ureterschienengrundkörpers. Dadurch ist erfindungsgemäß gewährleistet, dass das distale Ende des Ureterschienengrundkörpers nicht oder nicht übermäßig in die Einführkomponente eindringen kann. Damit kann zuverlässig verhindert werden, dass es zu Schwierigkeiten beim Lösen der beiden Elemente voneinander kommt.

**In** weiteren vorteilhaften Ausgestaltungen der erfindungsgemäßen Applikationsvorrichtung kann die Ausnehmung in der Einführkomponente in dem den ersten Rohrabschnitt zugewandten Endbereich mindestens eine Schräge und/oder mindestens eine Stufe aufweisen, derart, dass sich ein Durchmesser der Ausnehmung im Bereich der Schräge oder Stufe vergrößert. Die genannte Schräge oder Stufe kann auch durch einen distalen Endbereich des in dem ersten, proximalen Rohrabschnitt der Einführkomponente angeordneten Tubus ausgebildet werden. Die Ausbildung einer derartigen Schräge oder Stufe erleichtert das Herausgleiten des Aufnahmekörpers aus der Ausnehmung im Falle des Herausziehens des Führungselements aus dem Ureterschienengrundkörper, der Einführkomponente und des Aufnahmekörpers.

**In** einer weiteren vorteilhaften Ausgestaltung der erfindungsgemäßen Applikationsvorrichtung ist in einem proximalen Endbereich des ersten, proximalen Rohrabschnitts der Einführkomponente mindestens eine Kerbe oder randliche Ausnehmung zur Aufnahme und Führung des Verbindungselements ausgebildet ist. Beispielsweise werden in der Kerbe die Enden eines fadenoder drahtförmigen Verbindungselements zusammengeführt und zumindest teilweise gehalten. Vorteilhafterweise wird durch eine derartige Konstruktion verhindert, dass die Uretherschiene beziehungsweise das distale Ende des Ureterschienengrundkörpers sich unbeabsichtigt von dem proximalen Ende der Einführkomponente lösen kann.

In einer weiteren vorteilhaften Weiterbildung der Erfindung ist der zumindest eine Verbindungsbereich zwischen einer Freigabestellung, in welcher die wenigstens zwei Rohrabschnitte mittels des zumindest einen Verbindungsbereichs relativ zueinander verkippt sind und dadurch die Ausnehmung zur Freigabe des Aufnahmekörpers zumindest bereichsweise aufgeweitet ist, und einer Haltestellung, in welcher die Ausnehmung zum Halten des Aufnahmekörpers im Vergleich zur Freigabestellung verengt ist, verformbar. Dies ist von Vorteil, da somit durch das Verformen des Verbindungsbereichs von der Haltestellung in die Freigabestellung ein vereinfachtes Herausgleiten des Aufnahmekörpers aus der Ausnehmung ermöglicht ist. Während also der Aufnahmekörper in der Haltestellung verliersicher in der Ausnehmung gehalten werden kann, kann der Aufnahmekörper in der Freigabestellung auf besonders einfache Weise aus der Ausnehmung herausgeführt werden. Infolge des Verformens des Verbindungsbereichs von der Haltestellung in die Freigabestellung können jeweilige Mittelachsen der Rohrabschnitte zueinander verkippen und dadurch einen Winkel miteinander einschließen. In unverkipptem Zustand können die jeweiligen Mittelachsen der Rohrabschnitte parallel oder koaxial zueinander orientiert sein. Der zumindest eine Verbindungsbereich kann insbesondere elastisch zwischen der Haltestellung und der Freigabestellung verformbar sein.

In einer weiteren vorteilhaften Weiterbildung der Erfindung ist der zumindest eine Verbindungsbereich bei mittels der Einführkomponente gehaltener Ureterschiene sowie bei in die wenigstens zwei Rohrabschnitte eingeführtem Führungselement anhand des Führungselements in der Haltestellung haltbar, wobei eine elastische Verformung des zumindest einen Verbindungsbereichs von dessen Haltestellung in dessen Freigabestellung mittels des eingeführten Führungselements blockierbar ist. Dies ist von Vorteil, da dementsprechend allein durch Lösen des Führungselements von der Einführkomponente die elastische Verformung des Verbindungsbereichs von dessen Haltestellung in dessen Freigabestellung bewirkt werden kann. Dementsprechend kann durch das Lösen des Führungselements von der Einführkomponente auch gleichzeitig das Lösen der Ureterschiene, also des Ureterschienengrundkörpers und des Aufnahmekörpers, von der Einführkomponente unterstützt werden.

So kann beispielsweise der zumindest eine Verbindungsbereich in der Haltestellung anhand des biegeweichen Führungselements mit einer mechanischen Vorspannung beaufschlagt werden. Durch Herausführen des biegeweichen Führungselements aus dem ersten Rohrabschnitt kann die mechanische Vorspannung aufgehoben werden, wodurch der sich Verbindungsbereich selbstständig und elastisch von der Haltestellung in die Freigabestellung verformen kann.

Das eingeführte Führungselement kann somit die elastische Verformung des Verbindungsbereichs von dessen Haltestellung in dessen Freigabestellung unterbinden. Das eingeführte Führungselement kann dementsprechend den Verbindungsbereich in dessen Haltestellung gespannt halten.

In einer weiteren vorteilhaften Ausgestaltung der erfindungsgemäßen Applikationsvorrichtung ist das Verbindungselement bei an der Einführkomponente gehaltener Ureterschiene zumindestens teilweise an einer äußeren Oberfläche des proximalen Endes der Einführkomponente geführt. Eine derartige Anordnung des Verbindungselements ist vorteilhafterweise konstruktiv einfach herzustellen.

In einer weiteren vorteilhaften Ausgestaltung der erfindungsgemäßen Applikationsvorrichtung umfasst diese eine Montagehülse, wobei die Montagehülse derart ausgebildet ist, dass ein Innendurchmesser der Montagehülse gleich oder größer einem Außendurchmesser der Einführkomponente ist. Eine derartige Montagehülse dient zur Fixierung des Aufnahmekörpers in der Ausnehmung der Einführkomponente. Insbesondere vor einem Hindurchführen des Führungselements durch die entsprechenden Durchlassöffnungen der Einführkomponente und des Aufnahmekörpers kann so der Aufnahmekörper in der Ausnehmung sicher gehalten beziehungsweise fixiert werden.

Ein zweiter Aspekt der Erfindung betrifft eine Einführkomponente für eine Applikationsvorrichtung gemäß dem ersten Aspekt der Erfindung. Die im Zusammenhang mit der erfindungsgemäßen Applikationsvorrichtung gemäß dem ersten Aspekt der Erfindung vorgestellten Merkmale sowie deren Vorteile gelten entsprechend für die erfindungsgemäße Einführkomponente gemäß dem zweiten Aspekt der Erfindung und umgekehrt.

Ein dritter Aspekt der Erfindung betrifft eine Applikationseinrichtung, welche eine Applikationsvorrichtung gemäß dem ersten Aspekt der Erfindung sowie das biegeweiche Führungselement umfasst, an welchem die Ureterschiene sowie die Einführkomponente aufgenommen sind. Die im Zusammenhang mit der erfindungsgemäßen Applikationsvorrichtung gemäß dem ersten Aspekt der Erfindung und der erfindungsgemäßen Einführkomponente gemäß dem zweiten Aspekt der Erfindung vorgestellten Merkmale sowie deren Vorteile gelten entsprechend für die erfindungsgemäße Applikationseinrichtung gemäß dem dritten Aspekt der Erfindung und umgekehrt. Das biegeweiche Führungselement kann insbesondere durch jeweilige Durchgangsöffnungen des Ureterschienengrundkörpers und des Aufnahmekörpers, sowie durch die Einführkomponente geführt sein.

Weitere Merkmale der Erfindung ergeben sich aus den Ansprüchen, den Ausführungsbeispielen sowie anhand der Zeichnungen. Die vorstehend in der Beschreibung genannten Merkmale und Merkmalskombinationen sowie die nachfolgend in den Ausführungsbeispielen genannten Merkmale und Merkmalskombinationen sind nicht nur in der jeweils angegebenen Kombination, sondern auch in anderen Kombinationen verwendbar, ohne den Rahmen der Erfindung zu verlassen.

Dabei zeigt:
- Fig. 1: eine schematische Perspektivansicht einer Applikationseinrichtung, welche eine Applikationsvorrichtung und ein biegeweiches Führungselement umfasst, an welchem eine Ureterschiene sowie eine Einführkomponente der Applikationsvorrichtung geführt verschiebbar sind;
- Fig. 2: eine schematische Seitenansicht der Applikationseinrichtung, wobei einander entgegengesetzte Bewegungsrichtungen gezeigt sind, entlang welchen die Ureterschiene anhand der Einführkomponente translatorisch und relativ zu dem biegeweichen Führungselement verlagerbar ist und wobei einander entgegengesetzte Drehrichtungen gezeigt sind, in welche die Ureterschiene anhand der Einführkomponente relativ zu dem biegeweichen Führungselement drehbar ist;
- Fig. 3a-e: jeweils schematische Draufsichten auf ein Harnsystem eines Patienten, anhand welchen das Einführen des biegeweichen Führungselements in Richtung einer Niere des Harnsystems, eine anschließende Platzierung der Ureterschiene über das Führungselement mittels der Einführkomponente, die Entfernung des Führungselements und der Einführkomponente sowie eine gewünschte Lage der Ureterschiene bei deren bestimmungsgemäßem Gebrauch erkennbar sind;
- Fig. 4: eine schematische Perspektivansicht der Einführkomponente, wobei sich ein Verbindungsbereich der Einführkomponente in einer Freigabestellung befindet;
- Fig. 5: eine schematische Seitenansicht der Einführkomponente;
- Fig. 6: eine schematische Perspektivansicht eines distalen Grundkörperendes eines Ureterschienengrundkörpers der Ureterschiene sowie eines Aufnahmekörpers der Ureterschiene, welcher über ein Verbindungselement der Ureterschiene mit dem distalen Grundkörperende verbunden ist;
- Fig. 7: eine schematische Seitenansicht des distalen Grundkörperendes und des über das Verbindungselement mit dem distalen Grundkörperende verbundenen Aufnahmekörpers;
- Fig. 8a-c: schematische Darstellungen verschiedener Beispiele der Einführkomponente der nicht erfindungsgemäßen Applikationsvorrichtung; und
- Fig. 9a-b: schematische Darstellungen der erfindungsgemäßen Applikationsvorrichtung.

In den folgenden Figuren beziehen sich gleiche Bezugszeichen auf identische oder vergleichbare Merkmale der unterschiedlichen Ausführungsformen der Applikationsvorrichtung 10.

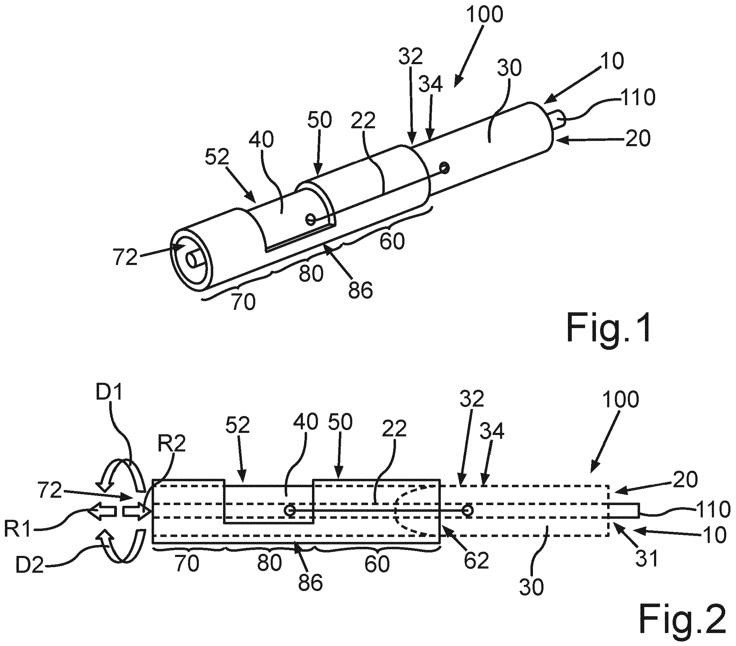

Fig. 1 und Fig. 2 zeigen jeweils verschiedenen Ansichten einer Applikationseinrichtung 100, welche eine Applikationsvorrichtung 10 sowie ein biegeweiches Führungselement 110 umfasst. An dem biegeweichen Führungselement 110, welches beispielsweise als Führungsdraht ausgebildet sein kann, kann einerseits eine Ureterschiene 20 und andererseits eine Einführkomponente 50 der Applikationsvorrichtung 10 angeordnet sein.

Die Ureterschiene 20 umfasst einen Ureterschienengrundkörper 30 und einen mittels wenigstens eines Verbindungselements 22 der Ureterschiene 20 mit dem Ureterschienengrundkörper 30 verbundenen Aufnahmekörper 40. Das wenigstens eine Verbindungselement 22 ist vorliegend biegeschlaff oder biegeweich ausgebildet und als Fadenelement ausgestaltet. Denkbar ist jedoch auch eine alternative Ausgestaltung des Verbindungselements 22 als Drahtelement. Man erkennt, dass das Verbindungselement 22 bei an der Einführkomponente 50 gehaltener Ureterschiene 20 zumindestens teilweise an einer äußeren Oberfläche eines proximalen Endes der Einführkomponente 50 geführt ist.

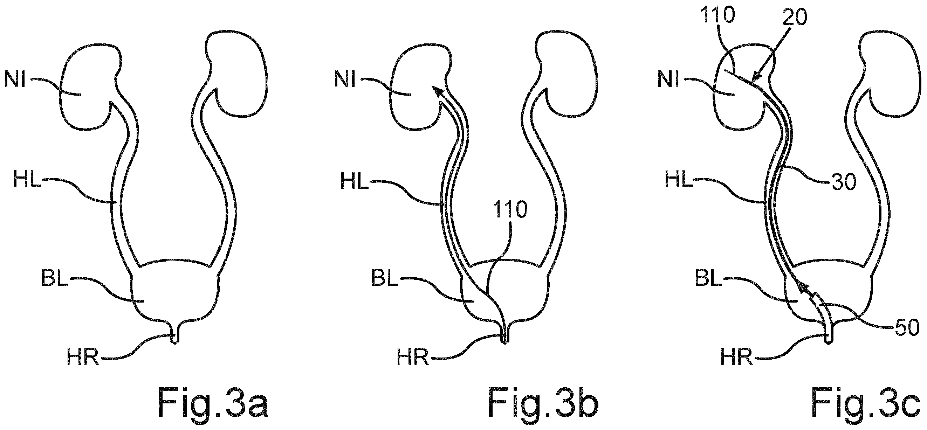

Die Einführkomponente 50 der Applikationsvorrichtung 10 dient zum Halten der Ureterschiene 20 und zum Führen der Ureterschiene 20 beispielsweise durch einen in Fig. 3a bis Fig. 3e erkennbaren Harnleiter HL eines Harnsystems eines Patienten entlang des biegeweichen Führungselements 110. Das Harnsystem ist in Fig. 3a ohne die Applikationseinrichtung 100 bzw. ohne die Applikationsvorrichtung 10 schematisch dargestellt und umfasst eine Harnröhre HR, eine Blase BL, Harnleiter HL und Nieren NI.

Um die Ureterschiene 20 in das Harnsystem einzusetzen, kann zunächst das biegeweiche Führungselement 110 (hier: Führungsdraht) über die Harnröhre HR, die Blase BL und einen der Harnleiter HL in eine der Nieren NI eingeführt werden, wie anhand von Fig. 3b erkennbar ist.

Fig. 3c zeigt, wie die Ureterschiene 20 im Anschluss daran mittels der Einführkomponente 50 entlang dem Führungselement 110 durch die Blase BL in Richtung des in der Zeichnungsebene linken Harnleiters HL und damit in Richtung der in der Zeichnungsebene linken Niere NI geschoben werden kann. Um dies zu ermöglichen, wird das Führungselement 110 einerseits in eine dem Ureterschienengrundkörper 30 zugeordnete Grundkörperdurchgangsöffnung 31 und andererseits in eine dem Aufnahmekörper 40 zugeordnete Aufnahmekörperdurchgangsöffnung 42 eingeführt. Zudem wird das Führungselement 110 durch jeweilige Rohrdurchgangsöffnungen 62, 72 der Einführkomponente 50 hindurchgeführt, während die Ureterschiene 20 an der Einführkomponente 50 gehalten ist.

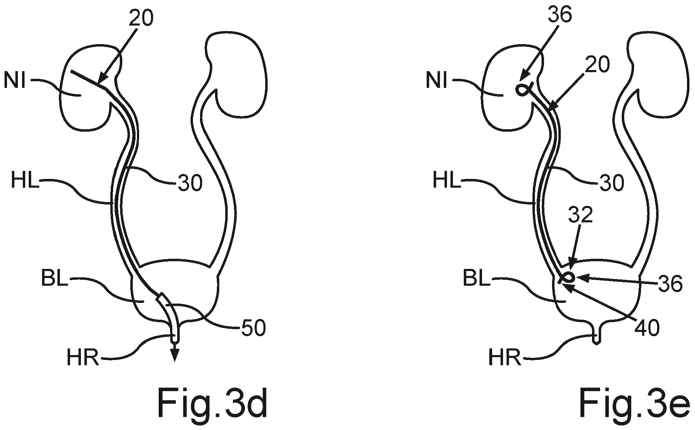

Anhand von Fig. 3d ist erkennbar, wie das Führungselement 110 entfernt wird, sobald die Ureterschiene 20 einerseits bereichsweise in die (linke) Niere NI eingeführt und andererseits bereichsweise in die Blase BL eingeführt ist. Fig. 3e zeigt schließlich eine vorbestimmte Lage der Ureterschiene 20 für deren bestimmungsgemäßen Gebrauch. Für den bestimmungsgemäßen Gebrauch der Ureterschiene 20 sind einander gegenüberliegende Grundkörperenden des Ureterschienengrundkörpers 30 in deren jeweiliger Grundstellung 36 jeweils j-förmig eingedreht, wodurch ein unerwünschtes Verrutschen der Ureterschiene 20 innerhalb des Harnsystems vermieden werden kann.

Die Ureterschiene 20 ist mittels der Einführkomponente 50 in einander entgegengesetzte Bewegungsrichtungen R1, R2 translatorisch innerhalb des Harnsystems verschiebbar. Die einander entgegengesetzten Bewegungsrichtungen R1, R2 sind in Fig. 2 durch einander entgegengesetzt gerichtete, gerade Pfeile verdeutlicht. Die Einführkomponente 50 ist zudem dazu ausgebildet, den Ureterschienengrundkörper 30 und den Aufnahmekörper 40 beim Verlagern entlang des Führungselements 110 und innerhalb des Harnsystems in den jeweiligen Bewegungsrichtungen R1, R2 zu führen. Darüber hinaus kann der Ureterschienengrundkörper 30 gemeinsam mit dem Aufnahmekörper 40 anhand der Einführkomponente 50 synchron und relativ zu dem biegeweichen Führungselement 110 in einander entgegengesetzte Drehrichtungen D1, D2 gedreht werden. Die Drehrichtungen D1, D2 sind in Fig. 2 durch einander entgegengesetzt gerichtete, gebogene Pfeile verdeutlicht.

Anhand von Fig. 1 und Fig. 2 ist zudem erkennbar, dass das wenigstens eine Verbindungselement 22 (hier: Faden) bei an der Einführkomponente 50 gehaltener Ureterschiene 20 mittels der Einführkomponente 50 gespannt ist.

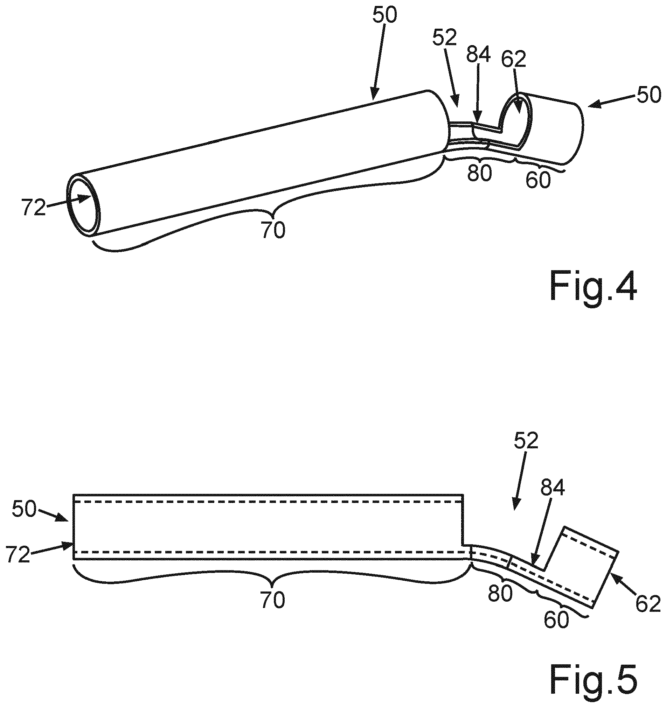

Durch Zusammenschau von Fig. 1, Fig. 4 und Fig. 5 ist besonders deutlich erkennbar, dass die Einführkomponente 50 eine Ausnehmung 52 zum Einführen und Halten des Aufnahmekörpers 40 umfasst. Die Ausnehmung 52 dient dabei als Aufnahme für den Aufnahmekörper 40. Die Einführkomponente 50 umfasst vorliegend zwei Rohrabschnitte, nämlich einen ersten Rohrabschnitt 60 und einen zweiten Rohrabschnitt 70, in welche das biegeweiche Führungselement 110 eingeführt werden kann, wie im Zusammenhang mit Fig. 3c bereits beschrieben wurde. Die Einführkomponente 50 umfasst des Weiteren einen Verbindungsbereich 80, welcher die Rohrabschnitte 60, 70 miteinander verbindet. Die beiden Rohrabschnitte 60, 70 und der Verbindungsbereich 80 begrenzen gemeinsam die Ausnehmung 52, wie insbesondere anhand von Fig. 4 und Fig. 5 erkennbar ist.

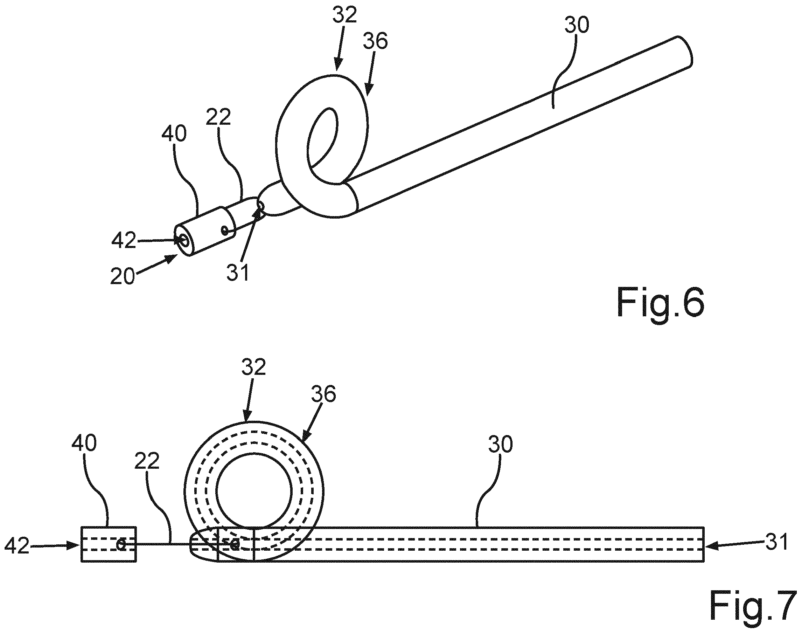

Der erste Rohrabschnitt 60 ist vorliegend als proximales Ende der Einführkomponente 50 ausgebildet, in welches - bei an der Einführkomponente 50 gehaltener Ureterschiene 20 (siehe Fig. 1) - ein dem Aufnahmekörper 40 zugewandtes, distales Grundkörperende 32 des Ureterschienengrundkörpers 30 bereichsweise eingeführt ist. Das Führungselement 110, welches sowohl durch die Einführkomponente 50, als auch durch den Aufnahmekörper 40 und durch den Ureterschienengrundkörper 30 hindurchgeführt ist, hält das bereichsweise eingeführte distale Grundkörperende 32 in einer Einführstellung 34 gespannt, insbesondere gestreckt. Darüber hinaus kann auch der erste Rohrabschnitt 60, in welchen das distale Grundkörperende 32 eingeführt ist, dazu beitragen, das distale Grundkörperende 32 gespannt zu halten. Der erste Rohrabschnitt 60 kann also das Halten des distalen Grundkörperendes 32 - bei an der Einführkomponente 50 gehaltener Ureterschiene 20 - in der Einführstellung 34 zumindest unterstützen. Das distale Grundkörperende 32 ist dazu ausgebildet, sich selbstständig von der Einführstellung 34 in die Grundstellung 36, welche in Fig. 3e und besonders deutlich in Fig. 6 und Fig. 7 erkennbar ist, des distalen Grundkörperendes 32 zu verformen und sich dadurch selbständig aus dem ersten Rohrabschnitt 60 heraus zu bewegen. In der Grundstellung 36 des distalen Grundkörperendes 32 ist Letzteres im Vergleich zur Einführstellung 34 entspannt. Durch Zusammenschau von Fig. 2, in welcher die Einführstellung 34 des distalen Grundkörperendes 32 besonders deutlich erkennbar ist, und Fig. 7, welche die Grundstellung 36 des distalen Grundkörperendes 32 zeigt, ist erkennbar, dass das distale Grundkörperende 32 in dessen Grundstellung 36 und im Gegensatz zur Einführstellung 34 eingedreht ist und dadurch eine größere radiale Erstreckung aufweist, als in der Einführstellung 34. Durch die Grundstellung 36 wird das unerwünschte Verrutschen des Ureterschienengrundkörpers 30 und damit der gesamten Ureterschiene 20 innerhalb des Harnsystems vermieden.

Das distale Grundkörperende 32 ist dazu ausgebildet, bei dessen (selbständigem) Herausbewegen aus dem ersten Rohrabschnitt 60 und durch das Verformen von der Einführstellung 34 in die Grundstellung 36 den Aufnahmekörper 40 über das Verbindungselement 22 aus der Ausnehmung 52 heraus zu ziehen. Hierzu kann das biegeweiche Führungselement 110 zunächst zumindest aus dem ersten Rohrabschnitt 60 und damit aus der ersten Rohrdurchgangsöffnung 62 herausgezogen werden, wodurch sich das distale Grundkörperende 32 elastisch und damit selbständig von der Einführstellung 34 in die Grundstellung 36 verformt und sich dadurch selbständig aus dem ersten Rohrabschnitt 60 heraus bewegt. Während des Verformens kann sich das distale Grundkörperende 32 an dem ersten Rohrabschnitt 60 abstützen und über das Verbindungselement 22 den Aufnahmekörper 40 aus der Ausnehmung 52 herausziehen. Das distale Grundkörperende 32 ist also dazu ausgebildet, bei dessen Ausführen aus dem ersten Rohrabschnitt 60 und durch das Verformen von der Einführstellung 34 in die Grundstellung 36 den Aufnahmekörper 40 über das Verbindungselement 22 aus der Ausnehmung 52 heraus zu ziehen.

Um das Herausziehen des Aufnahmekörpers 40 aus der Ausnehmung 52 zu erleichtern, ist der Verbindungsbereich 80 zwischen einer in Fig. 4 und Fig. 5 gezeigten Freigabestellung 84, in welcher die beiden Rohrabschnitte 60, 70 mittels des Verbindungsbereichs 80 relativ zueinander verkippt sind und dadurch die Ausnehmung 52 zur Freigabe des Herausziehens des Aufnahmekörpers 40 bereichsweise aufgeweitet ist, und einer in Fig. 1 in Fig. 2 gezeigten Haltestellung 86, in welcher die Ausnehmung 52 zum Halten des Aufnahmekörpers 40 im Vergleich zur Freigabestellung 84 verengt ist, elastisch verformbar.

Solange das biegeweiche Führungselement 110 (Führungsdraht) durch die beiden Rohrdurchgangsöffnungen 62, 72 der jeweiligen Rohrabschnitte 60, 70 der Einführkomponente 50 sowie durch die Grundkörperdurchgangsöffnung 31 und die Aufnahmekörperdurchgangsöffnung 42 geführt ist, verhindert das biegeweiche Führungselement 110 die elastische Verformung des Verbindungsbereichs 80 von dessen Haltestellung 86 in dessen Freigabestellung 84 und hält insbesondere den Verbindungsbereich 80 in der Haltestellung 86 vorgespannt. Sobald das biegeweiche Führungselement 110 jedoch zumindest aus der Grundkörperdurchgangsöffnung 31 und der ersten Rohrdurchgangsöffnung 62 vollständig herausgezogen ist, wirkt das biegeweiche Führungselement 110 der elastischen Verformung des Verbindungsbereichs 80 von dessen Haltestellung 86 in dessen Freigabestellung 84 nicht mehr entgegen, wodurch sich der Verbindungsbereich 80 selbstständig elastisch von der Haltestellung 86 in die Freigabestellung 84 verformen kann. Durch das hierdurch hervorgerufene Verkippen der beiden Rohrabschnitte 60, 70 wird die Ausnehmung 52 aufgeweitet, wie besonders deutlich in Fig. 4 und Fig. 5 erkennbar ist.

Somit wird also der Verbindungsbereich 80 bei mittels der Einführkomponente 50 gehaltener Ureterschiene 20 sowie bei in die Rohrabschnitte 60, 70 eingeführtem Führungselement 110 anhand des Führungselements 110 in der Haltestellung 86 vorgespannt gehalten, wobei die elastische Verformung des Verbindungsbereichs 80 von dessen Haltestellung 86 in dessen Freigabestellung 84 mittels des eingeführten Führungselements 110 blockiert werden kann, solange das Führungselement 110 insbesondere in die erste Rohrdurchgangsöffnung 62 des ersten Rohrabschnitts 60 und in die Grundkörperdurchgangsöffnung 31 des Ureterschienengrundkörpers 30 eingeführt ist.

Zusammenfassend ermöglicht die Einführkomponente 50 die Bewegung der Ureterschiene 20 (also des Ureterschienengrundkörpers 30 und des Aufnahmekörpers 40, welcher über das Verbindungselement 22 mit dem Ureterschienengrundkörper 30 gekoppelt ist) die Drehbewegungen D1, D2 sowie die translatorischen Bewegungen entlang der unterschiedlichen Bewegungsrichtungen R1, R2 relativ zu dem biegeweichen Führungselement 110 (Führungsdraht). Somit ist eine Positionierung der Ureterschiene 20 durch Vorziehen und Zurückziehen sowie durch Rotation der Ureterschiene 20 bei deren Einsetzen in das Harnsystem ermöglicht.

Die Einführkomponente 50, welche auch als Einführhilfe bezeichnet werden kann, dient also zum Positionieren der Ureterschiene 20, wobei die Einführkomponente 50 keine zusätzliche Mechanik benötigt und insbesondere keine speziellen Bewegungen erforderlich sind, um die Ureterschiene 20 nach deren Positionierung von der Einführkomponente 50 zu lösen. Aufgrund der speziellen Ausgestaltung der Einführkomponente 50 reicht es aus, das biegeweiche Führungselement 110 aus der Ureterschiene 20 und aus der Einführkomponente 50 herauszuziehen, um dadurch das Lösen der Ureterschiene 20 von der Einführkomponente 50 zu bewirken.

Die Verbindung zwischen der Einführkomponente 50 (Pusher) und der Ureterschiene 20 kann insbesondere selbstständig ohne extra dafür vorgesehene Bewegung beim Herausziehen des biegeweichen Führungselements 110 gelöst werden.

Die Verbindung zwischen der Ureterschiene 20 und der Einführkomponente 50 kann insbesondere durch eine federnde Bewegung des distalen Grundkörperendes 32 von dessen Einführstellung 34 in dessen Grundstellung 36 sowie durch eine weitere federnde Bewegung des Verbindungsbereichs 80 von dessen Haltestellung 86 in dessen Freigabestellung 84 gelöst werden, wobei der Verbindungsbereich 80 dem proximalen Ende der Einführkomponente 50 zugeordnet sein kann.

Der Verbindungsbereich 80 erfährt beim Einführen des biegeweichen Führungselements 110 zunächst eine reversible Formänderung, wobei der Verbindungsbereich 80 dadurch gespannt und von der Freigabestellung 84 in die Haltestellung 86 verformt wird. **In** der Haltestellung 86 ist ein besserer Sitz des Aufnahmekörpers 40 in der Ausnehmung 52 der Einführkomponente 50 gegeben, als in der Freigabestellung 84. Die entgegengesetzte Verformung des Verbindungsbereichs 80 von der Haltestellung 86 zurück in die Freigabestellung 84 kann durch das Herausziehen des biegeweichen Führungselements 110 zumindest aus der Grundkörperdurchgangsöffnung 31 und der ersten Rohrdurchgangsöffnung 62 bewirkt werden. In der Freigabestellung 84 kann im Vergleich zur Haltestellung 86 ein vereinfachtes Entfernen des Aufnahmekörpers 40 aus der (in der Freigabestellung 84 des Verbindungsbereichs 80 aufgeweiteten) Ausnehmung 52 erfolgen. Das distale Grundkörperende 32 erfährt durch das Einführen des biegeweichen Führungselements 110 in die Grundkörperdurchgangsöffnung 31 ebenfalls eine reversible Formänderung von der Grundstellung 36 in die Einführstellung 34. Die selbstständige, entgegengesetzte und damit rückläufige elastische Verformung des distalen Grundkörperendes 32 von der Einführstellung 34 in die Grundstellung 36 erfolgt durch das Herausziehen des Führungselements 110 zumindest aus der Grundkörperdurchgangsöffnung 31. Diese rückläufige elastische Verformung von der Einführstellung 34 in die Grundstellung 36 bewirkt das Herausziehen des Aufnahmekörpers 40 über das Verbindungselement 22 anhand des distalen Grundkörperendes 32.

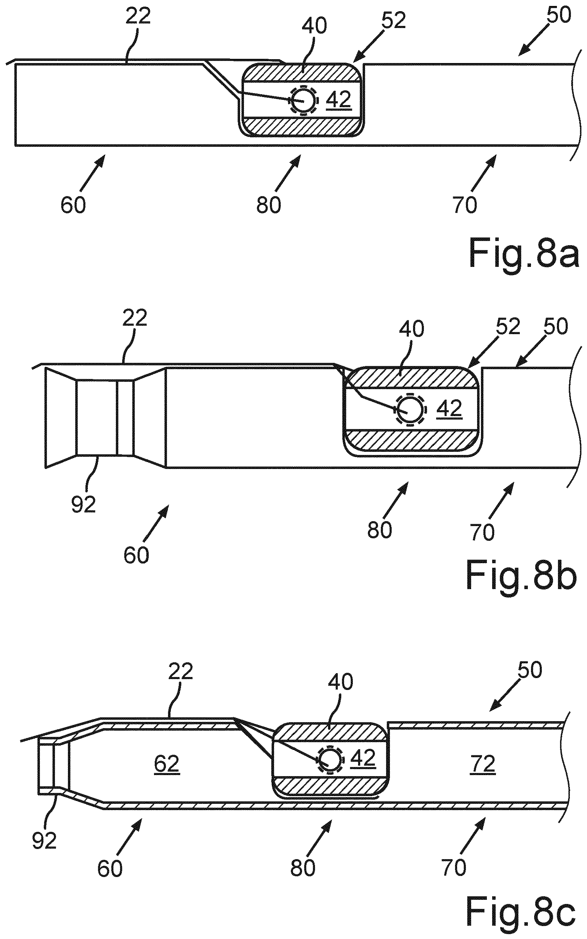

Die Fig. 8a bis 8c zeigen schematische Darstellungen verschiedener Ausführungsformen der Einführkomponente 50 der Applikationsvorrichtung 10. In Fig. 8a ist eine Einführkomponente 50 mit in die Ausnehmung 52 eingefügtem Aufnahmekörper 40 dargestellt. Man erkennt, dass die Ausnehmung 52 in dem den ersten Rohrabschnitt 60 zugewandten Endbereich eine Schräge 88 aufweist, derart, dass sich ein Durchmesser der Ausnehmung 52 im Bereich der Schräge 88 kontinuierlich nach außen hin vergrößert. Die Schräge 88 erleichtert ein Herausziehen des Aufnahmekörpers 40 mittels des mit dem distalen Ende 32 des Uretergrundkörpers 30 (hier nicht dargestellt) aus der Ausnehmung 52. Des Weiteren erkennt man, dass das Verbindungselement 22 fadenförmig ausgebildet ist und durch eine entsprechende Öffnung in dem Aufnahmekörper 40 verlaufend angeordnet ist.

In Fig. 8b ist eine weitere Ausführungsform der Einführkomponente 50 mit in die Ausnehmung 52 eingefügtem Aufnahmekörper 40 dargestellt. Man erkennt, dass der erste, proximale Rohrabschnitt 60 der Einführkomponente 50 in einem proximalen Endbereich eine Verengung 92 aufweist, wobei ein Innendurchmesser der Verengung 92 kleiner ist als ein Außendurchmesser des distalen Endes 32 des Ureterschienengrundkörpers 30 (hier nicht dargestellt). Des Weiteren erkennt man, dass das proximale Ende des proximalen Rohrabschnitts 60 trichterförmig ausgebildet ist. In Fig. 8c ist eine weitere Ausführungsform der Einführkomponente 50 mit in die Ausnehmung 52 eingefügtem Aufnahmekörper 40 dargestellt. Man erkennt, dass hier der erste, proximale Rohrabschnitt 60 der Einführkomponente 50 in einem proximalen Endbereich sich verjüngend ausgebildet ist, derart, dass der Innendurchmesser der Verengung 92 kleiner ist als der Außendurchmesser des distalen Endes 32 des Ureterschienengrundkörpers 30 (hier nicht dargestellt). Diese Ausgestaltungen ermöglichen es, dass das distale Ende 32 des Ureterschienengrundkörpers 30 nicht oder nicht übermäßig in die Einführkomponente eindringen kann und es so gegebenfalls zu Schwierigkeiten beim Lösen des distalen Endes 32 von der Einführungskomponente 50 kommt.

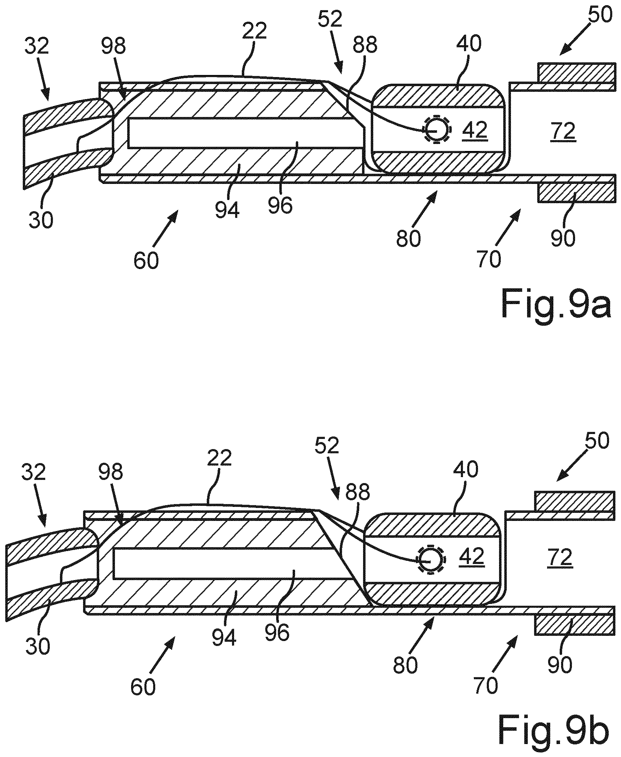

Die Fig. 9a und 9b zeigen schematische Darstellungen der Einführkomponente 50 der erfindungsgemäßen Applikationsvorrichtung 10.

In Fig. 9a ist eine Einführkomponente 50 mit in die Ausnehmung 52 eingefügtem Aufnahmekörper 40 dargestellt. Man erkennt, dass gemäß diesem Ausführungsbeispiel im ersten, proximalen Rohrabschnitt 60 der Einführkomponente 50 ein Tubus 94 angeordnet ist, wobei der Tubus 94 eine Tubusdurchgangsöffnung 96 aufweist, deren Durchmesser ungefähr gleich oder gleich einem Durchmesser der Durchgangsöffnung 42 des Aufnahmekörpers 40 ist. Zudem weist der Tubus 94 an seinem distalen Ende eine Schräge 88 auf, die zu einer Erweiterung des Durchmessers der Ausnehmung 52 führt. Die Schräge 88 verläuft dabei in einem 45°-Winkel über eine Länge von ungefähr der Hälfte des Durchmessers des Tubus 94. Das proximale Ende des Tubus 94 ist trichterförmig zur Aufnahme des distalen Endes 32 des Uretergrundkörpers 32 ausgebildet. Des Weiteren erkennt man, dass im proximalen Endbereich des proximalen Rohrabschnitts 60 eine Kerbe 98 zum Einführen und Halten des fadenförmigen Verbindungselements 22, insbesondere zur Aufnahme der beiden Enden des fadenförmigen Verbindungselements 22, aufweist. Die beiden Enden des fadenförmigen Verbindungselements 22 sind an dem distalen Ende 32 des Uretergrundkörpers 32 befestigt. Die Applikationsvorrichtung 10 gemäß diesem Ausführungsbeispiel weist zudem eine Montagehülse 90 auf, wobei die Montagehülse 90 derart ausgebildet ist, dass ein Innendurchmesser der Montagehülse 90 gleich oder größer einem Außendurchmesser der Einführkomponente 50 ist. Die Montagehülse 90 dient zur Fixierung des Aufnahmekörpers 40 in der Ausnehmung 52 der Einführkomponente 50 vor einem Hindurchführen des Führungselements 110 (hier nicht dargestellt) durch die entsprechenden Durchlassöffnungen 72, 96, 42 der Einführkomponente 50, des Tubus 94 und des Aufnahmekörpers 40. Der Aufnahmekörper 40 kann so in der Ausnehmung 52 sicher gehalten beziehungsweise fixiert werden.

Die in Fig. 9b dargestellte Ausführungsform der Einführkomponente 50 entspricht der in Fig. 9a beschriebenen und dargestellten Ausführungsform. Im Unterschied zu der zuvor beschriebenen Einführkomponente verläuft die im distalen Bereich des Tubus 94 ausgebildete Schräge 88 über den ganzen Durchmesser des Tubus 94. Die Schräge 88 dient wiederum zur Erweiterung des Durchmessers der Ausnehmung 52 in radialer Richtung nach Außen. Des Weiteren erkennt man, dass bei den in den Fig. 9a und 9b dargestellten Einführkomponenten 50 das proximale Ende zudem abgerundet ausgebildet ist. Diese Ausgestaltung führt zu einem vorteilhaften atraumatischen Spitzendesign der Einführkomponente 50.

## Patentansprüche

1. Applikationsvorrichtung (10), mit einer Ureterschiene (20), welche einen Ureterschienengrundkörper (30) und einen mittels wenigstens eines Verbindungselements (22) der Ureterschiene (20) mit dem Ureterschienengrundkörper (30) verbundenen Aufnahmekörper (40) umfasst, sowie mit einer Einführkomponente (50), zum Halten der Ureterschiene (20) und zum Führen der Ureterschiene (20) durch einen Harnleiter (HL) eines Patienten entlang eines biegeweichen Führungselements (110), wobei die Ureterschiene (20) mittels der Einführkomponente (50) in einander entgegengesetzte Bewegungsrichtungen (R1, R2) entlang dem biegeweichen Führungselement (110) verlagerbar ist und die Einführkomponente (50) dazu ausgebildet ist, den Ureterschienengrundkörper (30) und den Aufnahmekörper (40) beim Verlagern in den jeweiligen Bewegungsrichtungen (R1, R2) zu führen, und
die Einführkomponente (50) eine Ausnehmung (52) zum Einführen und Halten des Aufnahmekörpers (40) umfasst, wobei die Einführkomponente (50) wenigstens zwei Rohrabschnitte (60, 70) umfasst, in welche das biegeweiche Führungselement (110) einführbar ist, sowie zumindest einen, die wenigstens zwei Rohrabschnitte (60, 70) miteinander verbindenden, Verbindungsbereich (80) umfasst, wobei die wenigstens zwei Rohrabschnitte (60, 70) und der zumindest eine Verbindungsbereich (80) die Ausnehmung (52) begrenzen und ein erster Rohrabschnitt (60) der wenigstens zwei Rohrabschnitte (60, 70) als proximales Ende der Einführkomponente (50) ausgebildet ist, in welches zumindest bei an der Einführkomponente (50) gehaltener Ureterschiene (20) ein dem Aufnahmekörper (40) zugewandtes, distales Grundkörperende (32) des Ureterschienengrundkörpers (30) zumindest bereichsweise eingeführt ist, **dadurch gekennzeichnet, dass**
im ersten, proximalen Rohrabschnitt (60) der Einführkomponente (50) ein Tubus (94) angeordnet ist, wobei der Tubus (94) eine Tubusdurchgangsöffnung (96) aufweist, deren Durchmesser sich in Richtung des Aufnahmekörpers (40) verringert, derart, dass dieser ungefähr gleich oder gleich einem Durchmesser einer Durchgangsöffnung (42) des Aufnahmekörpers (40) ist oder der Tubus (94) eine Tubusdurchgangsöffnung (96) aufweist, deren Durchmesser ungefähr gleich oder gleich einem Durchmesser einer Durchgangsöffnung (42) des Aufnahmekörpers (40) ist und ein proximaler Endbereich des Tubus (94) trichterförmig zur Aufnahme des distalen Grundkörperendes (32) ausgebildet ist und/oder ein distaler Endbereich des Tubus (94) mindestens eine Schräge (88) und/oder mindestens eine Stufe aufweist, derart, dass sich ein Durchmesser der Ausnehmung (52) im Bereich der Schräge (88) oder Stufe vergrößert.

2. Applikationsvorrichtung (10) nach Anspruch 1,
**dadurch gekennzeichnet, dass**
der Aufnahmekörper (40) ferromagnetische Eigenschaften aufweist.

3. Applikationsvorrichtung (10) nach Anspruch 1 oder 2,
**dadurch gekennzeichnet, dass**
der Ureterschienengrundkörper (30) gemeinsam mit dem Aufnahmekörper (40) anhand der Einführkomponente (50) relativ zu dem biegeweichen Führungselement (110) drehbar ist.

4. Applikationsvorrichtung (10) nach einem der vorhergehenden Ansprüche, **dadurch gekennzeichnet, dass**
das wenigstens eine Verbindungselement (22) biegeschlaff oder biegeweich ausgebildet ist.

5. Applikationsvorrichtung (10) nach einem der vorhergehenden Ansprüche, **dadurch gekennzeichnet, dass**
das wenigstens eine Verbindungselement (22) bei an der Einführkomponente (50) gehaltener Ureterschiene (20) mittels der Einführkomponente (50) gespannt ist.

6. Applikationsvorrichtung (10) nach Anspruch 4 oder 5,
**dadurch gekennzeichnet, dass**
das wenigstens eine Verbindungselement (22) als Fadenelement oder als Drahtelement ausgebildet ist.

7. Applikationsvorrichtung (10) nach Anspruch 1,
**dadurch gekennzeichnet, dass**
das zumindest bereichsweise eingeführte distale Grundkörperende (32) bei an der Einführkomponente (50) gehaltener Ureterschiene (20) mittels des Führungselements (110) an dem ersten Rohrabschnitt in einer Einführstellung haltbar ist, wobei das distale Grundkörperende (32) dazu ausgebildet ist, sich bei von dem Ureterschienengrundkörper (30) gelöstem Führungselement (110) selbständig von der Einführstellung (34) in eine Grundstellung (36) des distalen Grundkörperendes (32) zu verformen und sich dadurch aus dem ersten Rohrabschnitt (60) heraus zu bewegen.

8. Applikationsvorrichtung (10) nach einem der Ansprüche 1 oder 7,
**dadurch gekennzeichnet, dass**
das distale Grundkörperende (32) dazu ausgebildet ist, bei dessen Herausbewegen aus dem ersten Rohrabschnitt (60) und durch das Verformen von der Einführstellung (34) in die Grundstellung (36) den Aufnahmekörper (40) über das wenigstens eine Verbindungselement (22) aus der Ausnehmung (52) heraus zu ziehen.

9. Applikationsvorrichtung (10) nach einem der Ansprüche 1 oder 7 bis 8, **dadurch gekennzeichnet, dass**
das der erste, proximale Rohrabschnitt (60) der Einführkomponente (50) in einem proximalen Endbereich eine Verengung (92) aufweist, wobei ein Innendurchmesser der Verengung (92) kleiner ist als ein Außendurchmesser des distalen Endes (32) des Ureterschienengrundkörpers (30) oder dass der erste, proximale Rohrabschnitt (60) der Einführkomponente (50) in einem proximalen Endbereich sich verjüngend ausgebildet ist, derart, dass der Innendurchmesser der Verengung (92) kleiner ist als der Außendurchmesser des distalen Endes (32) des Ureterschienengrundkörpers (30).

10. Applikationsvorrichtung (10) nach einem der Ansprüche 1 oder 7 bis 9, **dadurch gekennzeichnet, dass**
das der erste, proximale Rohrabschnitt (60) der Einführkomponente (50) eine erste Rohrdurchgangsöffnung (62) aufweist, deren Durchmesser sich in Richtung des Aufnahmekörpers (40) verringert, derart, dass dieser ungefähr gleich oder gleich einem Durchmesser einer Durchgangsöffnung (42) des Aufnahmekörpers (40) ist.

11. Applikationsvorrichtung (10) nach Anspruch 1,
**dadurch gekennzeichnet, dass**
die Ausnehmung (52) in dem den ersten Rohrabschnitt (60) zugewandten Endbereich mindestens eine Schräge (88) und/oder mindestens eine Stufe aufweist, derart, dass sich ein Durchmesser der Ausnehmung (52) im Bereich der Schräge (88) oder Stufe vergrößert.

12. Applikationsvorrichtung (10) nach einem der Ansprüche 1 oder 7 bis 11, **dadurch gekennzeichnet, dass**
das in einem proximalen Endbereich des ersten, proximalen Rohrabschnitts (60) der Einführkomponente (50) mindestens eine Kerbe (98) oder randliche Ausnehmung zur Aufnahme und Führung des Verbindungselements (22) ausgebildet ist.

13. Applikationsvorrichtung (10) nach einem der Ansprüche 1 oder 7 bis 12, **dadurch gekennzeichnet, dass**
der zumindest eine Verbindungsbereich (80) zwischen einer Freigabestellung (84), in welcher die wenigstens zwei Rohrabschnitte (60, 70) mittels des zumindest einen Verbindungsbereichs (80) relativ zueinander verkippt sind und dadurch die Ausnehmung (52) zur Freigabe des Aufnahmekörpers (40) zumindest bereichsweise aufgeweitet ist, und einer Haltestellung (86), in welcher die Ausnehmung (52) zum Halten des Aufnahmekörpers (40) im Vergleich zur Freigabestellung (84) verengt ist, verformbar ist.

14. Applikationsvorrichtung (10) nach Anspruch 13,
**dadurch gekennzeichnet, dass**
der zumindest eine Verbindungsbereich (80) bei mittels der Einführkomponente (50) gehaltener Ureterschiene (20) sowie bei in die wenigstens zwei Rohrabschnitte (60, 70) eingeführtem Führungselement (110) anhand des Führungselements (110) in der Haltestellung (86) haltbar ist, wobei eine elastische Verformung des zumindest einen Verbindungsbereichs (80) von dessen Haltestellung (86) in dessen Freigabestellung (84) mittels des eingeführten Führungselements (110) blockierbar ist.

15. Applikationsvorrichtung (10) nach einem der vorhergehenden Ansprüche, **dadurch gekennzeichnet, dass**
das Verbindungselement (22) bei an der Einführkomponente (50) gehaltener Ureterschiene (20) zumindestens teilweise an einer äußeren Oberfläche des proximalen Endes der Einführkomponente (50) geführt ist; oder **dadurch gekennzeichnet, dass** die Applikationsvorrichtung (10) eine Montagehülse (90) umfasst, wobei die Montagehülse (90) derart ausgebildet ist, dass ein Innendurchmesser der Montagehülse (90) gleich oder größer einem Außendurchmesser der Einführkomponente (50) ist.

16. Einführkomponente (50) für eine Applikationsvorrichtung (10) nach einem der Ansprüche 1 bis 15.

17. Applikationseinrichtung (100), welche eine Applikationsvorrichtung (10) nach einem der Ansprüche 1 bis 15 sowie ein biegeweiches Führungselement (110) umfasst, an welchem die Ureterschiene (20) sowie die Einführkomponente (50) aufgenommen sind.

## Claims

1. An application device (10) with a ureteral stent (20), which includes a ureteral stent base body (30) and a receiving body (40) connected to the ureteral stent base body (30) by means of at least one connection element (22) of the ureteral stent (20), as well as with an introduction component (50) for retaining the ureteral stent (20) and for guiding the ureteral stent (20) through a ureter (HL) of a patient along a flexible guide element (110), wherein the ureteral stent (20) is displaceable along the flexible guide element (110) in movement directions (R1, R2) opposite to each other by means of the introduction component (50), and the introduction component (50) is formed to guide the ureteral stent base body (30) and the receiving body (40) in displacing in the respective movement directions (R1, R2), and
the introduction component (50) includes a recess (52) for introducing and retaining the receiving body (40), wherein the introduction component (50) includes at least two pipe sections (60, 70), into which the flexible guide element (110) can be introduced, as well as at least one connection area (80) connecting the at least two pipe sections (60, 70) to each other, wherein the at least two pipe sections (60, 70) and the at least one connection area (80) delimit the recess (52), and a first pipe section (60) of the at least two pipe sections (60, 70) is formed as a proximal end of the introduction component (50), into which a distal base body end (32) of the ureteral stent base body (30) facing the receiving body (40) is introduced at least in certain areas at least with the ureteral stent (20) retained on the introduction component (50), **characterized in that**
a tube (94) is arranged in the first, proximal pipe section (60) of the introduction component (50), wherein the tube (94) comprises a tube passage opening (96), the diameter of which decreases towards the receiving body (40) such that it is approximately equal to or equal to a diameter of a passage opening (42) of the receiving body (40), or the tube (94) comprises a tube passage opening (96), the diameter of which is approximately equal to or equal to a diameter of a passage opening (42) of the receiving body (40), and a proximal end area of the tube (94) is formed funnel-shaped for receiving the distal base body end (32), and/or a distal end area of the tube (94) comprises at least one slant (88) and/or at least one step, such that a diameter of the recess (52) increases in the area of the slant (88) or step.

2. The application device (10) according to claim 1,
**characterized in that**
the receiving body (40) has ferromagnetic characteristics.

3. The application device (10) according to claim 1 or 2,
**characterized in that**
the ureteral stent base body (30) is rotatable in relation to the flexible guide element (110) in common with the receiving body (40) based on the introduction component (50).

4. The application device (10) according to any one of the preceding claims, **characterized in that**
the at least one connection element (22) is formed pliable or flexible.

5. The application device (10) according to any one of the preceding claims, **characterized in that**
the at least one connection element (22) is tensioned by means of the introduction component (50) with the ureteral stent (20) retained on the introduction component (50).

6. The application device (10) according to claim 4 or 5,
**characterized in that**
the at least one connection element (22) is formed as a thread element or as a wire element.

7. The application device (10) according to claim 1,
**characterized in that**
the distal base body end (32) introduced at least in certain areas can be retained on the first pipe section in an introduction position by means of the guide element (110) with the ureteral stent (20) retained on the introduction component (50), wherein the distal base body end (32) is formed to autonomously deform from the introduction position (34) into a basic position (36) of the distal base body end (32) with the guide element (110) detached from the ureteral stent base body (30) and to thereby move out of the first pipe section (60).

8. The application device (10) according to any one of claims 1 or 7, **characterized in that**
the distal base body end (32) is formed, upon movement thereof out of the first pipe section (60) and by the deformation from the introduction position (34) into the basic position (36), to extract the receiving body (40) out of the recess (52) via the at least one connection element (22).

9. The application device (10) according to any one of claims 1 or 7 to 8, **characterized in that**
the first, proximal pipe section (60) of the introduction component (50) comprises a constriction (92) in a proximal end area, wherein an internal diameter of the constriction (92) is smaller than an external diameter of the distal end (32) of the ureteral stent base body (30), or that the first, proximal pipe section (60) of the introduction component (50) is formed tapering in a proximal end area such that the internal diameter of the constriction (92) is smaller than the external diameter of the distal end (32) of the ureteral stent base body (30).

10. The application device (10) according to any one of claims 1 or 7 to 9, **characterized in that**
the first, proximal pipe section (60) of the introduction component (50) comprises a first pipe passage opening (62), the diameter of which decreases towards the receiving body (40), such that it is approximately equal to or equal to a diameter of a passage opening (42) of the receiving body (40).

11. The application device (10) according to claim 1,
**characterized in that**
the recess (52) comprises at least one slant (88) and/or at least one step in the end area facing the first pipe section (60), such that a diameter of the recess (52) increases in the area of the slant (88) or step.

12. The application device (10) according to any one of claims 1 or 7 to 11, **characterized in that**
at least one notch (98) or peripheral recess for receiving and guiding the connection element (22) is formed in a proximal end area of the first, proximal pipe section (60) of the introduction component (50).

13. The application device (10) according to any one of claims 1 or 7 to 12, **characterized in that**
the at least one connection area (80) is deformable between a release position (84), in which the at least two pipe sections (60, 70) are tilted in relation to each other by means of the at least one connection area (80) and thereby the recess (52) is expanded at least in certain areas for releasing the receiving body (40), and a retaining position (86), in which the recess (52) is constricted compared to the release position (84) for retaining the receiving body (40).

14. The application device (10) according to claim 13,
**characterized in that**
the at least one connection area (80) can be retained in the retaining position (86) based on the guide element (110) with the ureteral stent (20) retained by means of the introduction component (50) as well as with the guide element (110) introduced into the at least two pipe sections (60, 70), wherein an elastic deformation of the at least one connection area (80) from the retaining position (86) thereof into the release position (84) thereof can be blocked by means of the introduced guide element (110).

15. The application device (10) according to any one of the preceding claims, **characterized in that**
the connection element (22) is at least partially guided on an outer surface of the proximal end of the introduction component (50) with the ureteral stent (20) retained on the introduction component (50); or **characterized in that** the application device (10) includes a mounting sleeve (90), wherein the mounting sleeve (90) is formed such that an internal diameter of the mounting sleeve (90) is equal to or larger than an external diameter of the introduction component (50).

16. An introduction component (50) for an application device (10) according to any one of claims 1 to 15.

17. An application apparatus (100), which includes an application device (10) according to any one of claims 1 to 15 as well as a flexible guide element (110), on which the ureteral stent (20) as well as the introduction component (50) are received.

## Revendications

1. Dispositif d'application (10) ayant une endoprothèse urétérale (20) qui comprend un corps principal d'endoprothèse urétérale (30) et un corps de réception (40) relié au corps principal d'endoprothèse urétérale (30) au moyen d'au moins un élément de raccordement (22) de l'endoprothèse urétérale (20), ainsi qu'un composant d'introduction (50) destiné à maintenir l'endoprothèse urétérale (20) et à guider l'endoprothèse urétérale (20) à travers un uretère (HL) d'un patient le long d'un élément de guidage souple (110), l'endoprothèse urétérale (20) pouvant être déplacée au moyen du composant d'introduction (50) le long de l'élément de guidage souple (110) dans des directions de déplacement (R1, R2) mutuellement opposées et le composant d'introduction (50) étant formé pour guider le corps principal d'endoprothèse urétérale (30) et le corps de réception (40) lors du déplacement dans les directions de déplacement (R1, R2) respectives, et
le composant d'introduction (50) comprenant un évidement (52) destiné à introduire et maintenir le corps de réception (40), le composant d'introduction (50) comprenant au moins deux parties tubulaires (60, 70) dans lesquelles l'élément de guidage souple (110) peut être introduit, ainsi qu'au moins une zone de liaison (80) reliant les au moins deux parties tubulaires (60, 70) l'une à l'autre, les au moins deux parties tubulaires (60, 70) et la au moins une zone de liaison (80) délimitant l'évidement (52) et une première partie tubulaire (60) des au moins deux parties tubulaires (60, 70) étant formée comme une extrémité proximale du composant d'introduction (50), dans laquelle une extrémité distale de corps principal (32) du corps principal d'endoprothèse urétérale (30), dirigée vers le corps de réception (40) est introduite au moins dans certaines zones, au moins avec l'endoprothèse urétérale (20) maintenue sur le composant d'introduction (50), **caractérisé en ce que**
un tube (94) est disposé dans la première partie tubulaire proximale (60) du composant d'introduction (50), le tube (94) comportant une ouverture de passage de tube (96) dont le diamètre diminue en direction du corps de réception (40), de telle sorte que celui-ci est sensiblement égal ou égal à un diamètre d'une ouverture de passage (42) du corps de réception (40), ou le tube (94) comportant une ouverture de passage de tube (96) dont le diamètre est sensiblement égal ou égal à un diamètre d'une ouverture de passage (42) du corps de réception (40), et une zone d'extrémité proximale du tube (94) est formée en forme d'entonnoir pour recevoir l'extrémité distale de corps principal (32) et/ou une zone d'extrémité distale du tube (94) comportant au moins un biseau (88) et/ou au moins un gradin de telle sorte qu'un diamètre de l'évidement (52) augmente dans la zone du biseau (88) ou du gradin.

2. Dispositif d'application (10) selon la revendication 1,
**caractérisé en ce que**
le corps de réception (40) possède des propriétés ferromagnétiques.

3. Dispositif d'application (10) selon la revendication 1 ou 2,
**caractérisé en ce que**
le corps principal d'endoprothèse urétérale (30) peut tourner conjointement avec le corps de réception (40) par rapport à l'élément de guidage souple (110) au moyen du composant d'introduction (50).

4. Dispositif d'application (10) selon l'une des revendications précédentes,
**caractérisé en ce que**
le au moins un élément de raccordement (22) est formé de manière non rigide ou souple.

5. Dispositif d'application (10) selon l'une des revendications précédentes,
**caractérisé en ce que**
le au moins un élément de raccordement (22) est sollicité en tension au moyen du composant d'introduction (50) avec l'endoprothèse urétérale (20) maintenue sur le composant d'introduction (50).

6. Dispositif d'application (10) selon la revendication 4 ou 5,
**caractérisé en ce que**
le au moins un élément de raccordement (22) est formé comme un élément filamentaire ou un élément filaire.

7. Dispositif d'application (10) selon la revendication 1,
**caractérisé en ce que**
l'extrémité distale de corps principal (32), introduite au moins dans certaines zones, peut être maintenue dans une position d'introduction de la première partie tubulaire au moyen de l'élément de guidage (110) avec l'endoprothèse urétérale (20) maintenue sur le composant d'introduction (50), l'extrémité distale de corps principal (32) étant formée pour se déformer de façon autonome de la position d'introduction (34) à une position de base (36) de l'extrémité distale de corps principal (110) avec l'élément de guidage (110) détaché du corps principal d'endoprothèse urétérale (30), et pour ainsi sortir de la première partie tubulaire (60).

8. Dispositif d'application (10) selon l'une des revendications 1 ou 7,
**caractérisé en ce que**
l'extrémité distale de corps principal (32) est formée pour, lors d'un mouvement de celle-ci pour sortir de la première partie tubulaire (60) et par déformation de la position d'introduction (34) à la position de base (36), extraire le corps de réception (40) de l'évidement (52) par l'intermédiaire du au moins un élément de raccordement (22).

9. Dispositif d'application (10) selon l'une des revendications 1 ou 7 à 8,
**caractérisé en ce que**
la première partie tubulaire proximale (60) du composant d'introduction (50) comporte un rétrécissement (92) dans une zone d'extrémité proximale, un diamètre intérieur du rétrécissement (92) étant inférieur à un diamètre extérieur de l'extrémité distale (32) du corps principal d'endoprothèse urétérale (30), ou **en ce que** la première partie tubulaire proximale (60) du composant d'introduction (50) est formée en se rétrécissant dans une zone d'extrémité proximale de telle sorte que le diamètre intérieur du rétrécissement (92) est inférieur au diamètre extérieur de l'extrémité distale (32) du corps principal d'endoprothèse urétérale (30).

10. Dispositif d'application (10) selon l'une des revendications 1 ou 7 à 9,
**caractérisé en ce que**
la première partie tubulaire proximale (60) du composant d'introduction (50) comporte une première ouverture de passage tubulaire (62) dont le diamètre diminue en direction du corps de réception (40), de telle sorte qu'il est sensiblement égal ou égal à un diamètre d'une ouverture de passage (42) du corps de réception (40).

11. Dispositif d'application (10) selon la revendication 1,
**caractérisé en ce que**
l'évidement (52) dans la zone d'extrémité dirigée vers la première partie tubulaire (60) comporte au moins un biseau (88) et/ou au moins un gradin de telle sorte qu'un diamètre de l'évidement (52) augmente dans la zone du biseau (88) ou du gradin.

12. Dispositif d'application (10) selon l'une des revendications 1 ou 7 à 11,
**caractérisé en ce que**
dans une zone d'extrémité proximale de la première partie tubulaire proximale (60) du composant d'introduction (50) est formé au moins une encoche (98) ou un évidement de bord pour la réception et le guidage de l'élément de raccordement (22).

13. Dispositif d'application (10) selon l'une des revendications 1 ou 7 à 12,
**caractérisé en ce que**
la au moins une zone de liaison (80) est déformable entre une position de libération (84), dans laquelle les au moins deux parties tubulaires (60, 70) sont inclinées l'une par rapport à l'autre au moyen de la au moins une zone de liaison (80) et l'évidement (52) est ainsi élargi au moins dans certaines zones pour libérer le corps de réception (40), et une position de maintien (86) dans laquelle l'évidement (52) est rétréci par rapport à la position de libération (84) afin de maintenir le corps de réception (40).

14. Dispositif d'application (10) selon la revendication 13,
**caractérisé en ce que**
la au moins une zone de liaison (80) peut être maintenue dans la position de maintien (86) au moyen de l'élément de guidage (110) avec l'endoprothèse urétérale (20) maintenue au moyen du composant d'introduction (50), ainsi qu'avec l'élément de guidage (110) introduit dans les au moins deux parties tubulaires (60, 70), une déformation élastique de la au moins une zone de liaison (80) pouvant être bloquée de sa position de maintien (86) à sa position de libération (84) au moyen de l'élément de guidage (110) introduit.

15. Dispositif d'application (10) selon l'une des revendications précédentes,
**caractérisé en ce que**
l'élément de raccordement (22) est guidé au moins partiellement sur une surface extérieure de l'extrémité proximale du composant d'introduction (50) avec l'endoprothèse urétérale (20) maintenue sur le composant d'introduction (50) ; ou **caractérisé en ce que** le dispositif d'application (10) comprend une douille de montage (90), la douille de montage (90) étant formée de telle sorte qu'un diamètre intérieur de la douille de montage (90) est égal ou supérieur à un diamètre extérieur du composant d'introduction (50).

16. Composant d'introduction (50) pour un dispositif d'application (10) selon l'une des revendications 1 à 15.

17. Dispositif d'application (100) comprenant un dispositif d'application (10) selon l'une des revendications 1 à 15 ainsi qu'un élément de guidage souple (110) sur lequel l'endoprothèse urétérale (20) et le composant d'introduction (50) sont reçus.
